# EUROPÄISCHE PATENTSCHRIFT

(11) **EP 1 030 706 B1**
(45) Veröffentlichungstag und Bekanntmachung des Hinweises auf die Patenterteilung: **28.04.2004**
(21) Anmeldenummer: 98954389.7
(22) Anmeldetag: 09.10.1998
(51) Int. Cl.: A61M 25/02, A61M 39/10

(54) **VORRICHTUNG ZUM ZUFÜHREN VON FLÜSSIGKEITEN ZU EINEM PATIENTEN**
DEVICE FOR ADMINISTERING LIQUIDS TO A PATIENT
DISPOSITIF POUR ADMINISTRER DES SOLUTIONS LIQUIDES A UN PATIENT

(30) Priorität: 14.11.1997 DE 29720182 U
(43) Veröffentlichungstag der Anmeldung: 30.08.2000
(73) Patentinhaber: B. BRAUN MELSUNGEN AG, 34212 Melsungen (DE)
(72) Erfinder: GÖBEL, Udo, D-34212 Melsungen-Kirchhof (DE); FRIEDERICHS, Manfred, D-34212 Melsungen (DE); HARMS, Volker, D-34119 Kassel (DE); HEITMANN, Manfred, D-42781 Haan (DE); HINKEL, Tabea, D-34212 Melsungen (DE); KOCH, Karl-Heinz, D-34212 Melsungen (DE); OTTO, Hans-Joachim, D-34212 Melsungen (DE); SIEMON, Klaus, D-34327 Körle (DE); SIPPEL, Martin, D-34212 Melsungen (DE)
(74) Vertreter: Selting, Günther, Dipl.-Ing.
(86) Internationale Anmeldenummer: PCT/EP1998/006427
(87) Internationale Veröffentlichungsnummer: WO 1999/025414

(56) Entgegenhaltungen:
- FR-A- 2 722 414
- US-A- 3 794 032
- US-A- 4 170 995
- US-A- 5 137 519
- US-A- 5 188 609
- US-A- 5 468 230
- US-A- 5 690 617

## Beschreibung

Die Erfindung betrifft eine Vorrichtung zum Zuführen von Flüssigkeiten zu einem Patienten mit einem Zuführteil und einem Befestigungsteil. Bei dem Zuführteil, das von der Flüssigkeit durchströmbar ist, handelt es sich beispielsweise um einen Kanülenansatz, einen Katheteransatz oder einen Filter. Das Zuführteil ist durch das Befestigungsteil, beispielsweise auf der Haut des Patienten, an dessen Kleidung, an einem Krankenbett, an einem Infusionsständer oder anderen Gegenständen befestigt.

Üblicherweise werden Zuführteile mittels Klebeband am Körper eines Patienten befestigt, wobei die Klebebandstreifen über das Zuführteil verlaufen. Insbesondere bei Filtern führt dieses wahllose Überkleben dazu, daß der Filter nicht mehr einsehbar ist. Um festzustellen, ob der Filter verunreinigt ist, ist es erforderlich, die Klebestreifen von der Haut zu entfernen. Insbesondere wenn unterschiedliche Medikamente über einen Zugang dem Patienten zugeführt werden, ist es wichtig, den Filter häufig zu überprüfen, da unterschiedliche Medikamente miteinander reagieren und auskristallisieren können. Dies kann zu einer Blockade des Filters führen. Bei überklebten Filtern ist eine ununterbrochene Beobachtung des Filters nicht möglich, so daß diese weitgehend der Kontrolle des Arztes entzogen sind. Ferner dienen Filter bei Infusionen über einen langen Zeitraum zum Ausfiltern von Bakterien und Partikeln und müssen daher in bestimmten Zeitabständen ausgewechselt werden.

Kurzkatheter, die nahe der Einstichstelle auf der Hautoberfläche befestigt werden, weisen zum Teil Flügelansätze aus Kunststoff auf. Zur Fixierung der Kurzkatheter werden Klebebandstreifen über die Flügelansätze geklebt.

Eine derartige Fixierung von Filtern oder Kathetern an der Haut des Patienten schränkt die Bewegungsfreiheit des Patienten ein und kann bei Bewegungen des Patienten zu Verletzungen führen.

Aus US 5 456 671 ist die Befestigung eines Katheters mittels eines Klebepflasters an der Haut des Patienten bekannt. Hierzu weist das Klebepflaster ein erstes Kupplungsteil mit einem Längsschlitz auf, in den der Katheter einsetzbar ist. Trotzdem der Katheter von dem Kupplungsteil gelöst werden kann, ohne das Klebepflaster von der Haut des Patienten zu entfernen, ist der Katheter in dem Kupplungsteil fest gehalten, so daß die Bewegungsmöglichkeiten des Patienten weiterhin stark eingeschränkt sind. Des weiteren ist aus US-A-5 137 519 eine Vorrichtung mit den Merkmalen des Oberbegriffs des Anspruchs 1 bekannt.

Aufgabe der Erfindung ist es, eine Vorrichtung zum Zuführen von Flüssigkeiten zu Patienten zu schaffen, die auf einfache Weise am Patientenkörper oder an anderen Trägern befestigbar, einfach und schnell lösbar ist und die Bewegungsfreiheit des Patienten möglichst wenig einschränkt.

Die Lösung der Aufgabe erfolgt erfindungsgemäß durch die Merkmale des Patentanspruch 1.

Das Zuführteil ist mittels eines als Klebepflaster ausgebildeten Befestigungsteils fixiert, wobei das Befestigungsteil ein erstes Kupplungsteil aufweist, das mit einem an dem Zuführteil vorgesehenen zweiten Kupplungsteil lösbar zusammengreift. Erfindungsgemäß ist eines der beiden Kupplungsteile als Steckzapfen und das andere Kupplungsteil als runde Einstecköffnung ausgebildet. Durch die erfindungsgemäße Ausgestaltung der Kupplungsteile ist das Zuführteil gegenüber dem Klebepflaster verdrehbar. Dadurch wird die Bewegungsfreiheit des Patienten erhöht und das Risiko, daß beim Bewegen Verletzungen auftreten, verringert. Durch die Verdrehbarkeit des Zuführteils ist beispielsweise verhindert, daß eine in eine Vene eingeführte Nadel bei Bewegungen des Patienten die Vene durchsticht.

Das Klebepflaster wird auf die Hautoberfläche oder auf die Oberfläche eines Gegenstandes geklebt. Das Zuführteil kann auf einfache Weise mittels der Kupplungsteile am Befestigungsteil fixiert und wieder gelöst werden. Somit ist es nicht erforderlich, daß zur Befestigung des Zuführteils Klebebandstreifen über das Zuführteil verlaufen und dieses verdecken. Handelt es sich bei dem Zuführteil um einen Filter, so ist dieser gut einsehbar. Da der Filter mit dem Befestigungsteil über Kupplungsteile lösbar verbunden ist, kann der Filter zum Einsehen der Unterseite des Filters oder zum Auswechseln schnell und einfach von dem Befestigungsteil abgenommen werden, ohne daß das Befestigungsteil von der Hautoberfläche des Patienten oder von der Oberfläche eines Gegenstands abgezogen werden muß.

Die Kupplungsteile können so ausgebildet sein, daß das Fixieren des Zuführteils durch Klemmen eines der beiden Zuführteile in dem anderen Zuführteil lösbar fixiert ist. Alternativ können die Kupplungsteile mit einem Verriegelungsstift o.dgl. versehen sein, so daß ein Lösen der Verbindung nur durch Entriegeln der Kupplungsteile möglich ist.

Vorzugsweise sind die beiden Kupplungsteile als lösbare Rastelemente ausgebildet. Mindestens eines der beiden Rastelemente ist zumindest teilweise federnd ausgebildet und rastet in zusammengefügtem Zustand in das entsprechend ausgebildete andere Kupplungsteil ein. Zum Lösen der Rastverbindung können die Rastelemente so ausgebildet sein, daß der federnde Teil eines der Rastelemente von Hand zurückgedrückt werden muß, bevor das Zuführteil von dem Klebepflaster getrennt werden kann. Vorzugsweise sind die Rastelemente so ausgebildet, daß das Zuführteil durch Abziehen von dem Rastelement des Klebepflasters lösbar ist. Hierzu kann der federnde Teil des Rastelements bogenförmig ausgebildet sein, so daß der federnde Teil des Rastelements beim Abziehen des Zuführteils automatisch zurückgedrückt wird. Bei dieser Ausführung der Rastelemente ist es nicht erforderlich, den federnden Teil eines der Rastelemente von Hand zurückzudrücken.

Um eine möglichst große Bewegungsfreiheit des Patienten zu ermöglichen, weist der Steckzapfen einen zumindest teilweise kugelförmigen Bereich auf. Das Zuführteil kann bei einer derartigen Ausbildung des Steckzapfens nicht nur um die Längsachse des Steckzapfens gedreht werden, sondern bzgl. der Längsachse auch verkippt werden. Es handelt sich somit bei der Verbindung des Steckzapfens mit der Einstecköffnung um eine kugelgelenkartige Verbindung.

Um das Klebepflaster aus möglichst dünnem flexiblen Material herstellen zu können, damit es auch an nicht ebenen Oberflächen haftet, ist der Steckzapfen vorzugsweise an dem Pflaster und die Einstecköffnung in dem Zuführteil ausgebildet. Die Einstecköffnung kann als Sackloch oder als Durchgangsöffnung ausgebildet sein.

Nachfolgend wird die Erfindung anhand bevorzugter Ausführungsformen unter Bezugnahme auf die Zeichnungen näher erläutert.

Es zeigen:
- Fig. 1: eine schematische perspektivische Ansicht einer ersten bevorzugten Ausführungsform;
- Fig. 2: eine schematische Seitenansicht der ersten bevorzugten Ausführungsform;
- Fig. 3: eine schematische Draufsicht der ersten bevorzugten Ausführungsform;
- Fig. 4: eine schematische Draufsicht einer zweiten bevorzugten Ausführungsform; und
- Fig. 5: eine schematische Seitenansicht der zweiten bevorzugten Ausführungsform.

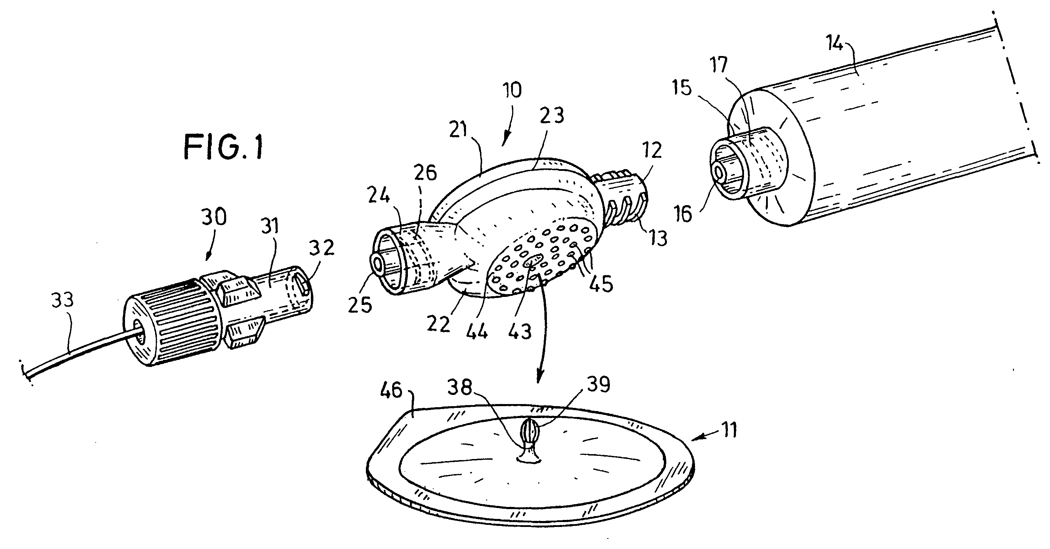

Die in den Fign. 1 bis 3 dargestellte Vorrichtung zum Zuführen von Flüssigkeiten zu einem Patienten weist ein Zuführteil in Form eines Filters 10 zum Filtern von Partikeln oder Bakterien und ein Klebepflaster 11 auf, mit dem der Filter 10 wie später beschrieben wird, lösbar zusammengreifen kann. Der Filter 10 weist einen zylindrischen Einlaßansatz 12 mit Außengewinde 13 auf. Mit dem Einlaßansatz 12 kann ein Spritzenzylinder 14 oder eine Zuführleitung, die mit einem Vorratsbehälter verbunden ist über einen zylindrischen Ansatz 15 dicht verbunden werden. An den Filter 10 oder ein anderes Zuführteil können somit sowohl Geräte zur Infusion als auch zur Injektion angeschlossen werden. Hierzu weist der Ansatz 15 einen konzentrisch zu dem Ansatz 15 angeordneten zylindrischen Stutzen 16 auf. Der Außendurchmesser des Stutzens 16 entspricht dem Innendurchmesser des Einlaßansatzes 12 des Filters 10. Um den Spritzenzylinder 14 fest mit dem Filter 10 verbinden zu können, weist der Ansatz 15 ein Innengewinde 17 auf, das mit dem Außengewinde 13 des Einlaßansatzes 12 zusammengreift.

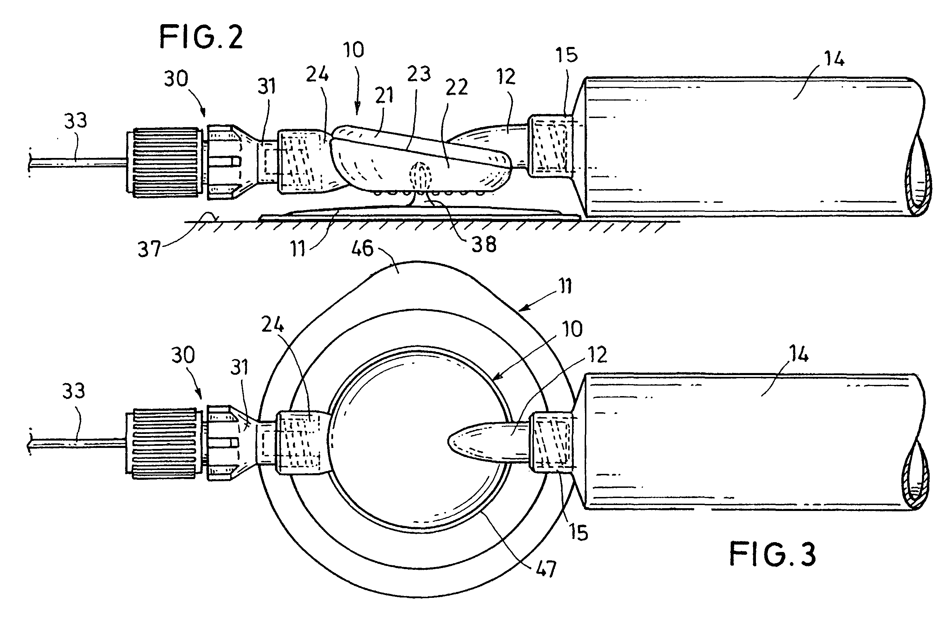

Der Einlaßansatz 12 des Filters 10 ist mit einem oberen Filterteil 21 verbunden, so daß die Flüssigkeit durch den Spritzenzylinder 14 in den oberen Filterteil 21 gelangt (Fig. 2). Mit dem oberen Filterteil 21 ist ein unteres Filterteil 22 verbunden, wobei in der Trennebene der beiden Filterteile eine Filtermembran 23 angeordnet ist. Die Flüssigkeit gelangt aus dem oberen Filterteil 21 durch die Filtermembran in das untere Filterteil 22. Das untere Filterteil 22 weist einen zylindrischen Auslaßansatz 24 auf, der entsprechend dem Ansatz 15 des Spritzenzylinders 14 einen konzentrischen Stutzen 25 und ein Innengewinde 26 aufweist. Der Auslaßansatz 24 des Filters 10 ist mit einem Kanülenansatz 30 verbindbar. Hierzu weist der Kanülenansatz 30 ein zylindrisches Ende 31 auf, dessen Außendurchmesser dem Innendurchmesser des Auslaßansatzes 24 des Filters 10 entspricht. An dem zylindrischen Ende 31 des Kanülenansatzes 30 ist ein Vorsprung 32 oder ein Außengewinde vorgesehen, der bzw. das in das Innengewinde 26 des Auslaßansatzes 24 des Filters 10 eingreift, so daß der Filter 10 mit dem Kanülenansatz 30 fest und dicht verbunden ist. Der Kanülenansatz 30 ist mit einer Kanüle 33 verbunden, die zu einem Patienten führt.

Das Klebepflaster 11 ist an seiner Unterseite mit einem Klebefilm versehen, so daß es auf einer Haut 37 eines Patienten (Fig. 2) oder auf einer Oberfläche eines Gegenstandes, wie einem Patientenbett oder einem Brutkasten aufklebbar ist. Das aus flexiblem Material bestehende Klebepflaster 11 weist mittig einen Steckzapfen 38 auf, der, wenn das Klebepflaster 11 auf einer Oberfläche aufgeklebt ist, senkrecht von dieser absteht. Der Steckzapfen 38 ist mit einem verdickten Kopf versehen und weist an seinem Kopf elastisch federnde Elemente 39 auf, die aus der in Fig. 1 dargestellten Lage zusammengedrückt werden können, so daß der Kopf des Steckzapfens 38 schmaler wird. Sobald keine Druckkraft mehr auf die elastisch federnden Elemente 39 des Steckzapfens ausgeübt wird, gehen die elastisch federnden Elemente 39 selbsttätig wieder in die in Fig. 1 dargestellte Ausgangslage zurück.

Zur Befestigung des Filters 10 an dem Klebepflaster 11 weist der Filter 10 eine Einstecköffnung 43 auf. Die Einstecköffnung 43 ist als nach innen erweitertes Sackloch ausgebildet (Fig. 2). Somit kann durch ein Aufstecken des Filters 10 auf den Steckzapfen 38 der Filter 10 rastend fixiert werden. Durch die gebogene Ausbildung der federnden Elemente 39 und der entsprechenden Form der Einstecköffnung 43 kann der Filter 10 auf einfache Weise von dem Steckzapfen 38 abgezogen werden. Ein Lösen der Rastverbindung durch einen zusätzlichen Handgriff ist nicht erforderlich. Da die Einstecköffnung 43 rund ist, kann der Filter 10 gegenüber dem Pflaster 11 verdreht werden.

Aufgrund der runden Ausgestaltung der Einstecköffnung. 43 und des kugelförmigen verdickten Kopfes des Steckzapfens 38 kann der Filter 10 sowohl um die Längsachse des Steckzapfens 38 gedreht werden, als auch verkippt werden, so daß die Unterseite des Filters 10 nicht mehr parallel zur Oberfläche 37 ist. Dadurch ist die Bewegungsfreiheit des Patienten erhöht.

Wie in Fig. 2 dargestellt, ist die Tiefe der als Sackloch ausgebildeten Einstecköffnung 43 bezüglich der Länge des Steckzapfens 38 so gewählt, daß der Filter 10 im Abstand zu der Oberfläche 37 angeordnet ist. Sofern es sich bei der Oberfläche 37 um die Haut eines Patienten handelt, wird dadurch vermieden, daß der Filter 10 auf der Haut des Patienten Druckstellen hervorruft. Die Länge des Steckzapfens 38 kann ferner so gewählt werden, daß in Abhängigkeit des verwendeten Filters 10 und des Durchmessers der Zuführleitung 14 die Zuführleitung 14 auf der Oberfläche 37 aufliegt. Da der Filter 10 aufgrund der Ausgestaltung des Steckzapfens 38 verkippbar ist, können Abmessungsunterschiede zwischen dem Durchmesser der Zuführleitung 14 und der Länge des Steckzapfens 38 ausgeglichen werden. Die kugelgelenkartige Ausbildung der Verbindung zwischen dem Filter 10 und dem Klebepflaster 11 dient somit auch als Ausgleich, wenn nicht vollständig aufeinander abgestimmte Filter 10 und Zuführleitungen 14 verwendet werden. Wird an den Einlaßansatz 12 des Filters 10 kurzzeitig eine Spritze mit größerem Durchmesser angeschlossen, so kann der Filter 10 von dem Steckzapfen 38 des Klebepflasters 11 abgezogen und nach dem Zuführen der Flüssigkeit aus der Spritze wieder auf den Steckzapfen 38 aufgesteckt werden. Ein schmerzhaftes Entfernen von den Filter 10 befestigenden Klebestreifen ist nicht erforderlich.

Um den Filter 10 auch direkt auf der Hautoberfläche 37 eines Patienten ohne Verwendung des Klebepflasters 11 befestigen zu können, weist eine dem Klebepflaster 11 zugewandte Seite 44 Noppen 45 auf, so daß zwischen dem Filter 10 und der Hautoberfläche 37 Luft frei zirkulieren kann. Die halbkugelförmigen und singulär angeordneten Noppen 45 sind hautfreundlicher als Rippen und dgl. an der Filterunterseite und vermeiden Druckstellen oder Verletzungen durch scharfe Kanten.

Zur Erleichterung des Abziehens des Klebepflasters 11 weist das Klebepflaster 11 eine nicht mit Klebstoff beschichtete Lasche 46 auf.

Wie aus Fig. 3 ersichtlich ist, hat das Klebepflaster 11 einen größeren Durchmesser als der Filter 10. Beim Aufstecken des Filters 10 auf den Steckzapfen 38 des Klebepflasters 11 ist der Steckzapfen 38 sowie die Einstecköffnung 43 von oben nicht sichtbar. Da der Steckzapfen 38 und die Einstecköffnung 43 jeweils mittig an dem Klebepflaster 11 bzw. dem Filter 12 vorgesehen sind, ist das Pflaster mit einem Kreis 47 bedruckt, dessen Durchmesser geringfügig größer als der des Filters 10 ist, so daß der Filter 10 auch von oben einfach gegenüber dem Klebepflaster 11 zentriert werden kann. Dadurch ist das Aufstecken des Filters 10 auf den Steckzapfen 38 des Klebepflasters 11 erheblich vereinfacht.

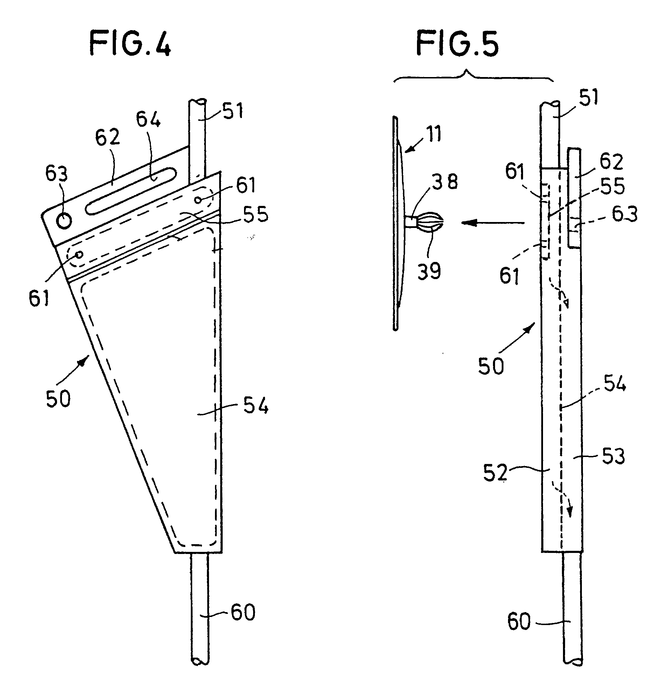

In den Fign. 4 und 5 ist eine zweite Ausführungsform einer Zuführvorrichtung dargestellt. Ein Filter 50 wird beispielsweise zur Infusion von Flüssigkeiten über lange Zeiträume eingesetzt und kann am Körper des Patienten, an dessen Kleidung oder an der Bettwäsche angebracht werden. Im dargestellten Ausführungsbeispiel ist der Filter 50 annähernd dreieckig und flach ausgebildet. Bei der Infusion muß sichergestellt sein, daß keine Verunreinigungen oder Gasbläschen mit der Infusionsflüssigkeit zum Patienten transportiert werden. Um sicherzustellen, daß der Filter 50 nicht falsch angeschlossen wird, ist der Filter 50 in etwa dreieckig ausgebildet, wobei die Spitze des Dreiecks in die Flußrichtung weist. Damit die Flußrichtung eindeutig erkennbar ist, kann der Filter auch trichterförmig u.dgl. ausgebildet sein.

Die Flüssigkeit gelangt über einen Zuführschlauch 51 in die in Fig. 5 linke Filterhälfte 52 des Filters 50. Die linke Filterhälfte 52 ist von der rechten Filterhälfte 53 durch eine hydrophile Membran 54 getrennt. Die hydrophile Membran 54 hat die Eigenschaft, daß bis zu einem relativ hohen Druck, der während der Infusion nicht erreicht wird, nur Flüssigkeiten und keine Gase durch die hydrophile Membran 54 gelangen können. Im oberen Bereich des Filters ist eine hydrophobe Membran 55 angeordnet, durch die nur Gase und keine Flüssigkeiten gelangen. Die hydrophobe Membran deckt zwei Öffnungen 61 ab, durch die Gase aus dem Filter 50 austreten. In die rechte Filterhälfte 53 kann durch die hydrophile Membran 54 nur Flüssigkeit gelangen, die durch einen Auslaßschlauch 60 in eine zu dem Patienten führende Kanüle fließt.

Zur Befestigung des Filters 50 an der Hautoberfläche eines Patienten oder an der Oberfläche eines Gegenstandes kann ebenfalls das anhand der ersten Ausführungsform beschriebene Klebepflaster 11 verwendet werden. Da das Vorsehen eines Sacklochs bei einem flachen Filter im Filterbereich schwierig ist, weist der Filter 50 einen Ansatz 62 auf, der mit dem oberen Bereich des Filters 50, an dem der Zuführschlauch 51 gehalten ist, fest verbunden ist. Der flache Ansatz 62 weist eine runde Einstecköffnung 63 auf, deren Innendurchmesser größer als der Durchmesser des Stiels des Steckzapfens 38 und kleiner als der Kopfdurchmesser des Steckzapfens 38 ist. Zur Befestigung des Filters 50 an dem Klebepflaster 11 wird der Filter 50 so auf den Steckzapfen 38 gesteckt, daß die federnde Elemente 39 des Steckzapfens 38 von der Einstecköffnung 63 zusammengedrückt werden und nach Durchtreten der Eintrittsöffnung 63 wieder in ihre ursprüngliche Form zurückfedern. Dadurch ist der Filter 50 mit einer lösbaren Rastverbindung an dem Klebepflaster 11 gehalten. Entsprechend dem Filter 10 der ersten Ausführungsform kann die Befestigung des Filters 50 durch einfaches Abziehen von dem Steckzapfen 38 wieder gelöst werden.

Um ein Anliegen des Filters 50 an der Hautoberfläche oder der Oberfläche des Gegenstands, an dem das Klebepflaster 11 befestigt ist, zu vermeiden, kann der Steckzapfen an dem dem Klebepflaster 11 zugewandten Ende einen Wulst aufweisen, dessen Durchmesser größer als die Einstecköffnung 63 ist. Ferner kann der Filter 50 einen weiteren Ansatz mit Einstecköffnung aufweisen, so daß der Filter 50 an mehreren Stellen mit einem Steckzapfen eines Klebepflasters verbindbar ist.

Zusätzlich weist der Ansatz 62 ein Langloch 64 auf. Durch das Langloch 64 kann ein Band gezogen werden, um den Filter 50 mit Hilfe des Bandes und einer an dem Band vorgesehenen Klemmvorrichtung an der Kleidung des Patienten zu befestigen. Durch das durch das Langloch 64 gezogene Band kann der Filter an einem Infusionsständer, an der Kleidung oder an einem anderen Gegenstand befestigt werden.

## Patentansprüche

1. Vorrichtung zum Zuführen von Flüssigkeiten zu einem Patienten, mit einem Zuführteil (10,50), das von der Flüssigkeit durchströmbar ist, und einem Klebepflaster (11) zum Fixieren des Zuführteiles (10,50), wobei das Klebepflaster (11) ein erstes Kupplungsteil (38) aufweist, welches mit einem an dem Zuführteil (10,50) vorgesehenen zweiten Kupplungsteil (43,63) lösbar zusammengreift,
wobei eines der beiden Kupplungsteile (38;43,63) als Steckzapfen (38) und das andere Kupplungsteil als runde Einstecköffnung (43,63) ausgebildet ist, **dadurch gekennzeichnet, daß** das Zuführteil (10,50) gegenüber dem Klebepflaster (11) um eine senkrecht zur Ebene des Klebepflasters verlaufende Achse verdrehbar ist.

2. Vorrichtung nach Anspruch 1, **dadurch gekennzeichnet, daß** die beiden Kupplungsteile (38;43,63) als Rastelemente ausgebildet sind.

3. Vorrichtung nach Anspruch 1 oder 2, **dadurch gekennzeichnet, daß** das Zuführteil (10,50) durch den Steckzapfen (38) in Abstand zu einer Oberfläche (37) gehalten wird, an der das Klebepflaster (11) aufgeklebt ist.

4. Vorrichtung nach einem der Ansprüche 1 bis 3, **dadurch gekennzeichnet, daß** der Steckzapfen (38) an dem Klebepflaster (11) und die Einstecköffnung (43,63) an dem Zuführteil (10,50) vorgesehen ist.

5. Vorrichtung nach einem der Ansprüche 1 bis 4, **dadurch gekennzeichnet, daß** das Klebepflaster (11) eine nicht klebende Lasche (46) aufweist.

6. Vorrichtung nach einem der Ansprüche 1 bis 5, **dadurch gekennzeichnet, daß** das Zuführteil (50) zusätzlich ein Langloch (64) zum Befestigen des Zuführteils (50) mittels eines Bandes aufweist.

7. Vorrichtung nach einem der Ansprüche 1 bis 6, **dadurch gekennzeichnet, daß** die dem Klebepflaster (11) zugewandte Oberfläche (44) des Zuführteils (10) mit Noppen (45) versehen ist.

8. Vorrichtung nach einem der Ansprüche 1 bis 7, **dadurch gekennzeichnet, daß** der Steckzapfen (38) einen kugelförmigen verdickten Kopf aufweist, der eine Kippung des Zuführteils (10,50) zuläßt.

## Claims

1. Device for administering liquids to a patient's body, comprising an administering portion (10,50) through which liquid can flow, and an adhesive plaster (11) for fixing the administering portion (10,50), wherein the adhesive plaster (11) comprises a first coupling member (38) which releasably engages with a second coupling member (43,63) provided on the administering portion (10,50), wherein one of the two coupling members (38;43,63) is configured as plug-in pin (38) and the other coupling member as round insertion opening (43,63)
**characterized in that**
the administering portion (10,50) is rotatable relatively to the adhesive plaster (11) about an axis extending vertically to the plane of the adhesive plaster.

2. Device according to claim 1, **characterized in that** the two coupling members (38;43,63) are configured as snap elements.

3. Device according to claim 1 or 2, **characterized in that** the plug-in pin (38) keeps the administering portion (10,50) at a distance to a surface (37) on which the adhesive plaster (11) is stuck.

4. Device according to one of claims 1 to 3, **characterized in that** the plug-in pin (38) is provided on the adhesive plaster (11) and the insertion opening (43,63) on the administering portion (10,50).

5. Device according to one of claims 1 to 4, **characterized in that** the adhesive plaster (11) comprises a non-adhesive flap (46).

6. Device according to one of claims 1 to 5, **characterized in that** the administering portion (50) further comprises an oblong hole (64) for fastening the administering portion (50) with a tape.

7. Device according to one of claims 1 to 6, **characterized in that** the surface (44) of the administering portion (10) facing the adhesive plaster (11) is provided with knops (45).

8. Device according to one of claims 1 to 7, **characterized in that** the plug-in pin (38) comprises a spherical thickened head which allows the administering portion (10,50) to be tilted.

## Revendications

1. Dispositif pour amener des liquides à un patient, comprenant une pièce d'amenée (10, 50) pouvant être traversée par le liquide, et un timbre adhésif de support (11) pour fixer la pièce d'amenée (10, 50), le timbre adhésif de support (11) comportant une première partie d'accouplement (38) qui vient en prise démontable avec une seconde partie d'accouplement (43, 63) prévue sur la pièce d'amenée (10, 50), l'une des deux parties d'accouplement (38 ; 43, 63) étant réalisée sous forme d'un tenon enfichable (38) et l'autre partie d'accouplement sous forme d'une ouverture d'enfichage ronde (43, 63),
**caractérisé en ce que** la pièce d'amenée (10, 50) peut tourner par rapport au timbre adhésif de support (11), autour d'un axe s'étendant perpendiculairement au plan du timbre adhésif de support.

2. Dispositif selon la revendication 1, **caractérisé en ce que** les deux parties d'accouplement (38 ; 43, 63) sont réalisés sous forme d'éléments d'encliquetage.

3. Dispositif selon la revendication 1 ou 2, **caractérisé en ce que** la pièce d'amenée (10, 50) est maintenue par le tenon enfichable (38), à distance d'une surface (37) sur laquelle est collée le timbre adhésif de support (11).

4. Dispositif selon l'une des revendications 1 à 3, **caractérisé en ce que** le tenon enfichable (38) est prévu sur le timbre adhésif de support (11) et l'ouverture d'enfichage (43, 63) sur la pièce d'amenée (10, 50).

5. Dispositif selon l'une des revendications 1 à 4, **caractérisé en ce que** le timbre adhésif de support (11) comporte une languette (46) non adhésive.

6. Dispositif selon l'une des revendications 1 à 5, **caractérisé en ce que** la pièce d'amenée (50) présente en supplément un trou oblong (64) pour la fixation de la pièce d'amenée (50) au moyen d'une bande.

7. Dispositif selon l'une des revendications 1 à 6, **caractérisé en ce que** la surface (44) de la pièce d'amenée (10), qui est dirigée vers le timbre adhésif de support (11), est pourvue de protubérances (45).

8. Dispositif selon l'une des revendications 1 à 7, **caractérisé en ce que** le tenon enfichable (38) présente une tête renflée de forme sphérique, qui autorise un basculement de la pièce d'amenée (10, 50).
